Europäisches Patentamt

European Patent Office

Office européen des brevets

(19)

(11) Publication number: **0 274 147**

**A2**

(12) **EUROPEAN PATENT APPLICATION**

(21) Application number: **87202353.6**

(22) Date of filing: **27.11.87**

(51) Int. Cl.4: **C12P 33/00** , **C07J 1/00**

(30) Priority: **03.12.86 NL 8603083**

(43) Date of publication of application:
**13.07.88 Bulletin 88/28**

(84) Designated Contracting States:
**AT BE CH DE ES FR GB GR IT LI LU NL SE**

(71) Applicant: **Nederlandse Organisatie voor Toegepast Natuurwetenschappelijk Onderzoek TNO**
**J. van Stolberglaan 148**
**NL-2595 CL Den Haag(NL)**

(72) Inventor: **Kerkenaar, Antonius**
**Plaggewagen 12**
**NL-1261 KG Blaricum(NL)**
Inventor: **Hesselink, Paulus Gerhardus Maria**
**Klaas de Vriezestraat 7**
**NL-9741 AE Groningen(NL)**

(74) Representative: **van der Beek, George Frans et al**
**Nederlandsch Octrooibureau Johan de Wittlaan 15 P.O. Box 29720**
**NL-2502 LS 's-Gravenhage(NL)**

(54) **Process for preparing steroids.**

㉝ The invention relates to a process for preparing steroids by enzymatic or microbial conversion of other steroids, such as sterols, whereby undesired breakdown of the steroid nucleus is inhibited by the presence of 3-phenylpropyl or alkyl substituted morpholine or piperidine compounds, such as fenpropimorph, tridemorph and fenpropidine.

EP 0 274 147 A2

## Process for preparing steroids

The invention relates to a process for preparing steroids.

To an important extent the present industrial production of pharmaceutical steroid products is based on the raw material diosgenin, a sapogenin from dioscorea tubers. The production of said diosgenin is, however, insufficient to fulfil the requirement as raw material. A search has therefore been made for new raw materials and for new processes adapted to said raw materials in order to transform them into pharmacological steroid products or suitable precursors therefor. Cholesterol and cheap phytosterols such as sitosterol, campesterol and stigmasterol, which phytosterols are obtained as a mixture (approx. 6 : 3.5 : 0.5) from, inter alia, soya bean, are appropriate as suitable raw materials. As is indicated below, the selective microbial side-chain breakdown of these sterols mentioned as raw materials offers good possibilities for preparing desirable compounds such as androstene derivatives.

The microbial steriod conversion mentioned above is, however, characterized by two points:

a) steroids (sterols) are not natural substrates for microorganisms, which aspect leads to side reactions and loss of product; and

b) substrate and product are very sparingly soluble in water, which leads to retarded reactions.

In view of the continuous competitive battle between, on the one hand, the microbial conversion and, on the other hand, the organic chemical production methods, a microbial steroid transformation should be very specific, i.e. without side reactions or degradation of the sterol nucleus in order for it to be economically attractive.

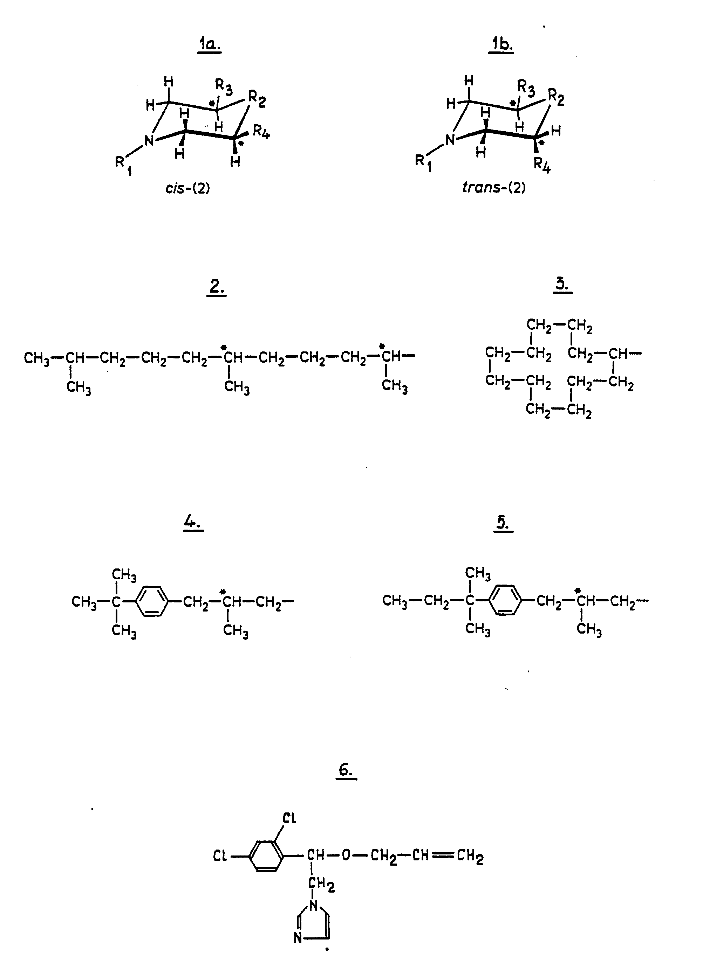

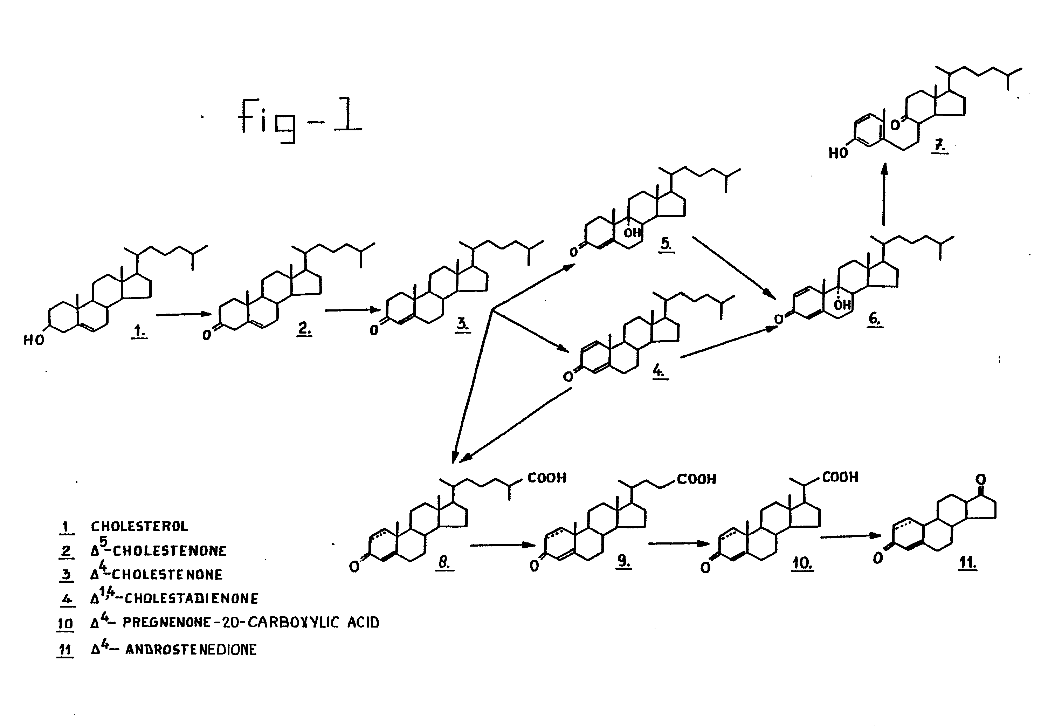

Inter alia, on the basis of the review entitled "Biotechnology, A Comprehensive Treatise in 8 Volumes", edited by H.J. Rehm and G. Reed, Vol. 6a, chapter 3 entitled "Sterols" it is reasonable to formulate the - scheme illustrated in Figure 1 for the breakdown route of steroids and sterols, which appears to be generally valid for the genera Arthrobacterium, Bacterium, Nocardia and Pseudomonas. In all probability, said breakdown route illustrated in Figure 1 is also followed in the genera Bacillus, Brevibacterium, Corynebacterium, Mycobacterium and Streptomyces, in view of the fact that in said genera identical reactions and reaction products are encountered during the steroid breakdown. In said Figure 1, the main route of the microbial conversion of cholesterol to the (precursors for) pharmacological steroid products is pregnenone carboxylic acid and androstenedione (1 - 2 - 3 (-4) - 8 - 9 - 10 - 11). The route for the nucleus degradation is also indicated (1 - 2 - 3 - 4,5-6 - 7).

More particularly, the steroid breakdown indicated in Figure 1 is divided into two parts: the steroid nucleus degradation and the side-chain breakdown. Both processes occur simultaneously and virtually independently of each other.

For preparing pharmacological steroid products from sterols (steroids) by means of bacteria, a nucleus breakdown is considered undesirable. After all after opening the B ring in the reaction 6-7 indicated in Figure 1, pharmacological steroid production is no longer possible so that at least this reaction stage should be blocked.

A selective side-chain breakdown in which no nucleus degradation occurs is indeed possible, according to the abovementioned review, with

a) mutants which have been blocked as regards the nucleus degradation;

b) structure-modified substrates; and

c) inhibiting compounds for the enzymes which are responsible for the nucleus degradation.

Re a). Although much research has already been done on finding the suitable mutants, this has not yet yielded any really good strains. The most promising strains are the Mycobacterium sp. NRRL-B 3683 and NRRL-B 3805. These strains have been produced by UV irradiation and exhibit the desired side-chain breakdown to androstene derivatives, the nucleus degradation also being largely blocked. In addition, the Corynebacteria ATCC 31455 and ATCC 31458 and the mutants derived therefrom are mentioned in this connection. The two abovementioned wildtype soil organisms are capable of growing on cholesterol and of converting it into androstene and pregnene compounds with degradation of the side chains. The Corynebacteria ATCC 31456, ATCC 31457, ATCC 31459 and ATCC 31460, which exhibit (virtually) no nucleus degradation, have been obtained therefrom by chemical mutagenesis with penicillin.

Re b). In relation to the structure-modified substrates it is reported that research has primarily been done into sterols with the following structure modifications or combinations thereof: sterol 3-$\beta$-esters, sterol 3-$\beta$-alkyl ethers, sterols with 3-$\beta$-nitrogen functions, 19-hydroxysterols, 6-$\beta$-19-oxidosterols and 5-chloro/bromosterols. A beneficial aspect of such modifications is often the enhanced solubility of the substrate, as a result of which the transport of substrate to the cells is increased. Reasonable results have been obtained on a laboratory scale by means of immobilized cells. An important disadvantage of such structure modifications is that a number of additional conversion stages are required, namely, first the stage of providing the substrates with a masking group and then the stage of ridding the products again of said masking groups. In addition, said stages are accompanied by additional washing and purification stages, as a result of which said production method has largely lost its attractiveness.

Re c). Fairly good results have been achieved in using enzyme inhibitors which are responsible for the nucleus degradation. The inhibition of the 9-α-hydroxylase complex, which contains some iron/sulphur enzymes, has, in particular, proved successful in this connection (see the reaction stages 3 - 4 and 5 - 6 in Figure 1). The inhibition was carried out by means of (lipophilic) chelating compounds for $Fe^{2+}$ ($\alpha,\alpha'$-dipyridyl, 8-hydroxy quinoline, 1,10-phenanthrolines), metal ions which are capable of replacing $Fe^{2}$ ($Ni^{2}$, $Co^{2}$, $Pb^{2}$), sulphydryl reagents ($SeO_3^{2-}$, $AsO_3^{2-}$) redox dyestuffs (methylene blue, resazurin) and rhamnolipids. The steroid production can be increased by adding antibiotics since the cell-wall or cell-membrane permeability increases. Strains which are much used for this method are: Arthrobacter simplex, Mycobacterium phlei, Nocardia coralina and Nocardia erythropolis.

In this last method, cell immobilization is used on a large scale, as a result of which it is possible, inter alia, to employ two-liquid-phase systems such as toluene/water and carbontetrachloride/water, in which case virtually no cell damage occurs.

Although good results have already been achieved on a laboratory scale, such as 70% conversion of cholesterol into pregnane derivatives by means of Nocardia erythropolis (see Process Biochemistry, January/February 1983, pages 19-21: Microbial Catalysts for Steroid Transformations - Part 2), these methods do not yet appear capable of competing with production methods already in existence.

The invention now offers the possibility of directly and selectively influencing and controlling the enzyme cholesterol oxidase, both intracellularly and extracellularly and also in purified form, in order to prevent isomerization.

The invention therefore makes a contribution to improving the enzymatic or microbial conversion of cholesterol and the abovementioned phytosterols into suitable androstene derivatives. More particularly, the invention relates to a fundamentally new process for preparing androst-4-ene-3,17-dione and androsta-1,4-diene-3,17-dione, optionally as a mixture with androst-5-ene-3,17-dione and androsta-1,5-diene-3,17-dione by means of a microbial conversion of cholesterol, sitosterol, campesterol or stigmasterol, or a mixture of one or more of said starting substances in which the microbial conversion is carried out in the presence of a 3-phenylpropylamine compound or alkylamine compound having the formula 1a or 1b in which $R_1$ represents a group having the formulae 2, 3, 4 or 5, $R_2$ represents an -O-or -CH₂-link, $R_3$ represents an H atom or $CH_3$ group, and $R_4$ represents an H atom or $CH_3$ group, or related compounds having the same mechanism of action, namely the inhibition of $\Delta^8 \xrightarrow{} \Delta^7$ isomerization, $\Delta^{14}$ reduction, cycloeucalenolobtusifoliol isomerization in fungi and higher plants and $\Delta^5 \xrightarrow{} \Delta^4$ isomerization (see Modern Selective Fungicides, edited by H. Lyr, chapter 12, A. Kerkenaar, Mechanism of action of morpholine fungicides, VEB Gustav Fischer Verlag, Jena, in press, and P. Benveniste, Sterol Biosynthesis, Ann Rev. Plant Physiol. 37 - (1986), 275-308).

The abovementioned 3-phenylpropylamine and alkylamine compounds having the formula 1a or 1b in which $R_1$ represents a group having the formula 2, 3, 4 or 5, $R_2$ represents -O-or -CH₂-, $R_3$ represents $-CH_3$ or -H, and $R_4$ represents $-CH_3$ or -H, have the following commercial names:

tridemorph: a compound having the formula 1a or 1b in which $R_1$ represents the group having the formula 2, $R_2$ represents -O-and $R_3$ and $R_4$ represent $-CH_3$,
which is: 4-(1,5,9-trimethyldecyl)-2,6-dimethylmorpholine;
dodemorph: a compound having the formula 1a or 1b in which $R_1$ represents the group having the formula 3, $R_2$ represents -O-and $R_3$ and $R_4$ represent $-CH_3$,
which is: 4-cyclododecyl-2,6-dimethylmorpholine;
fenpropimorph: a compound having the formula 1a or 1b, in which $R_1$ represents the group having the formula 4, $R_2$ represents -O-and $R_3$ and $R_4$ represent $-CH_3$,
which is: 4-[3-[4-(1,1-dimethylethyl)phenyl]-2-methylpropyl]-2,6-dimethylmorpholine;
amorolfine: a compound having the formula 1a or 1b in which $R_1$ represents the group having the formula 5, $R_2$ represents -O-and $R_3$ and $R_4$ represent $-CH_3$,
which is: 4-[3-[4-(1,1-dimethylpropyl)phenyl]-2-methylpropyl]-2,6-dimethylmorpholine;
fenpropidine: a compound having the formula 1a or 1b in which $R_1$ represents the group having the formula 4, $R_2$ represents -CH₂-and $R_3$ and $R_4$ represent -H,
which is:1-[3-[4-(1,1-dimethylethyl)phenyl]-2-methylpropyl]piperidine.

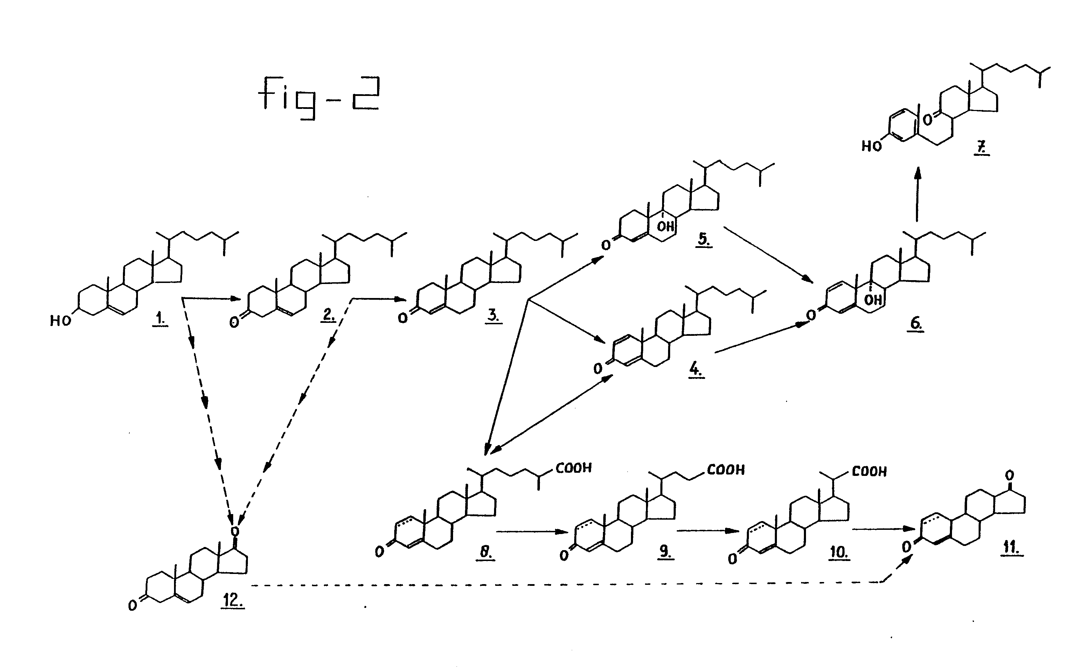

More particularly, the abovementioned 3-phenylpropylamine or alkylamine compounds inhibit the transition shown in Figure 1 of compound (2) to compound (3). A possible conversion route which is followed when the process according to the invention is applied is shown in Figure 2, the broken lines

3

representing the "3-phenylpropylamine or alkylamine compounds" route. The way is opened to a number of basically new methods of controlling and mastering microbial and enzymatic steroid conversions by the selective inhibition of the isomerization reaction, one of the activities of the cholesterol oxidase enzyme involved in this connection. The selective side-chain breakdown of sterols to androsta-1,4-diene-3,17-dione and androst-4-ene-3,17-dione is a good example thereof.

In the enzymatic steroid conversion according to the invention, use is made of microbial cholesterol oxidases in intact cells or in purified form. Such cholesterol oxidases are encountered, for example, in Nocardia, Corynebacterium, Arthrobacter, Mycobacterium, Pseudomonas, Schizophyllum, Brevibacterium, Rhodococcus and Streptomyces species.

Preferably, the enzymatic conversion according to the invention is carried out in the presence of a quantity of 0.001 - 10 g/l 3-phenylpropylamine or alkylamine compounds having the formula 1a or 1b in which $R_1$, $R_2$, $R_3$ and $R_4$ have the abovementioned meaning. Advantageously, fenpropimorph, tridemorph and fenpropidine are used.

The androst-4-ene-3,17-dione obtained in the process according to the invention is a valuable starting material in the preparation of pharmacologically active products. For example, androst-4-ene-3,17-dione can be converted into testosterone and other steroids with an androgenic action.

The process according to the invention is explained by reference to the examples below which should not be interpreted as restrictive.

## EXAMPLE I

Use of the sterol biosynthesis inhibitors fenpropimorph, tridemorph, fenpropidine and imazalil having formula 6 as potential inhibitors for purified cholesterol oxidases.

The cholesterol oxidases originated from Pseudomonas testosteroni and Nocardia erythropolis and were supplied respectively by Sigma (grade III enzyme, claimed $\beta$ activity of 3.3 U/mg) and Boehringer. NAD , cholesterol, $\Delta^5$-cholestenone, $\Delta^4$-cholestenone, pregnenolone and progesterone also originated from Sigma and were checked before use for purity by means of UV - VIS spectroscopy (Perkin-Elmer), and TLC and capillary GC (Packard: 436, 25 m CP Sil 5 CB column of Chrompack) as MO-TMS derivatives.

It is known that in the case of 3-hydroxy-5-ene steroids spontaneous isomerization of the C5 double bond to the C4 double bond does not occur. This will possibly not be true for 3-keto-5-ene steroids since a conjugated dienone which is more favourable from an energy point of view is formed in the C5 to C4 isomerization. The formation of said dienone (3-keto-4 -ene structure) can readily be observed by UV spectroscopy at 239nm, the molar extinction coefficient being approximately 15,000 - 20,000 l/mol.cm, whereas a 3-keto-5 -ene structure has an extinction coefficient of 300 - 400 l/mol.cm at said wavelength.

From tests made by the Applicant, non-enzymatic isomerization of the double bond from the C5 to C4 position has not in fact been observed in 3-keto-5 -ene structures after 72 hours at 30°C in 0.1-1% solutions (w/v) in a buffer with a pH between 7 and 9 and isopropanol mixtures. It is therefore assumed that the formation of cholest-4-ene-3-one under these conditions is an enzymatically catalysed reaction which is blocked by means of the inhibitors according to the invention.

In order to be able to investigate this selective blocking of cholesterol oxidase activity, enzyme assays were then developed in which the cholesterol oxidase from P. testosteroni and N. erythropolis functioned optimally. This resulted in the following reaction conditions:
- for P. testosteroni:
    0.875 ml of 100 mM tris, pH 8.85, containing 0.025% Triton X - 100,
    0.125 ml of isopropanol,
    0.050 mg of NAD ,
    0.020 mg of cholesterol oxidase, 3.3 U/mg,
    0.020 mg of pregnenolone.
Measurement at 25°C in a 1 ml cell at a wavelength of 340 (oxidation) or 239 nm (isomerization + oxidation). The molar extinction coefficient of progesterone is 18,506 l/mol.cm at 239 nm and the molar extinction coefficient of NADH is 6,230 l/mol.cm at 340 nm.
- for N. erythropolis:
    0.750 ml of 50 mM sodium phosphate buffer, pH 7.0, containing 0.025% Triton X - 100,
    0.250 ml of isopropanol,
    0.020 mg of cholesterol or $\Delta^5$-cholestenone,
    0.020 mg cholesterol oxidase U/mg, after desalination via a Sephadex G 25 PD 10 column.
Measurement at 30°C in a 1 ml cell at a wavelength of 239 nm. The molecular extinction coefficient of $\Delta^4$-

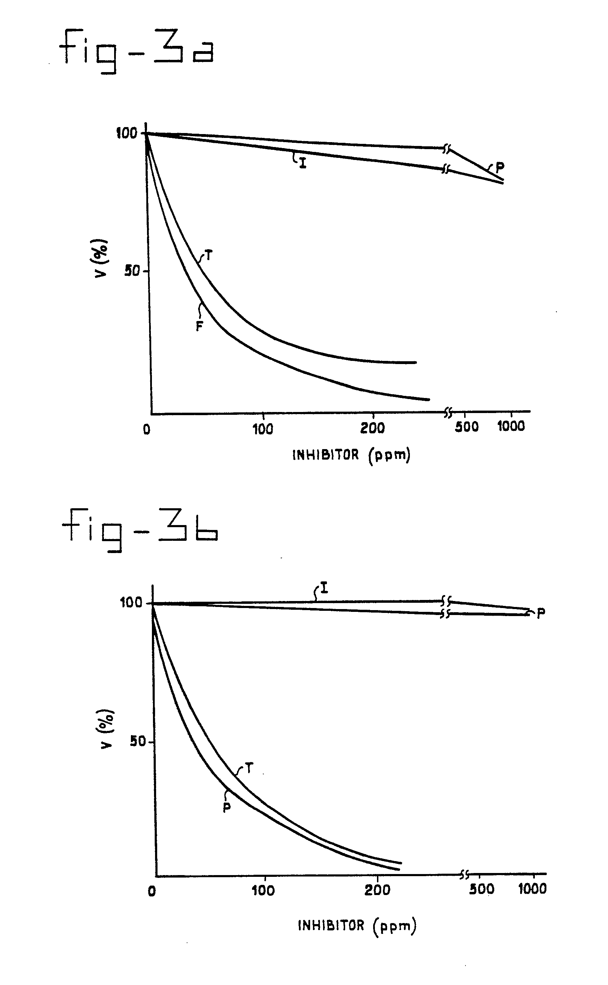

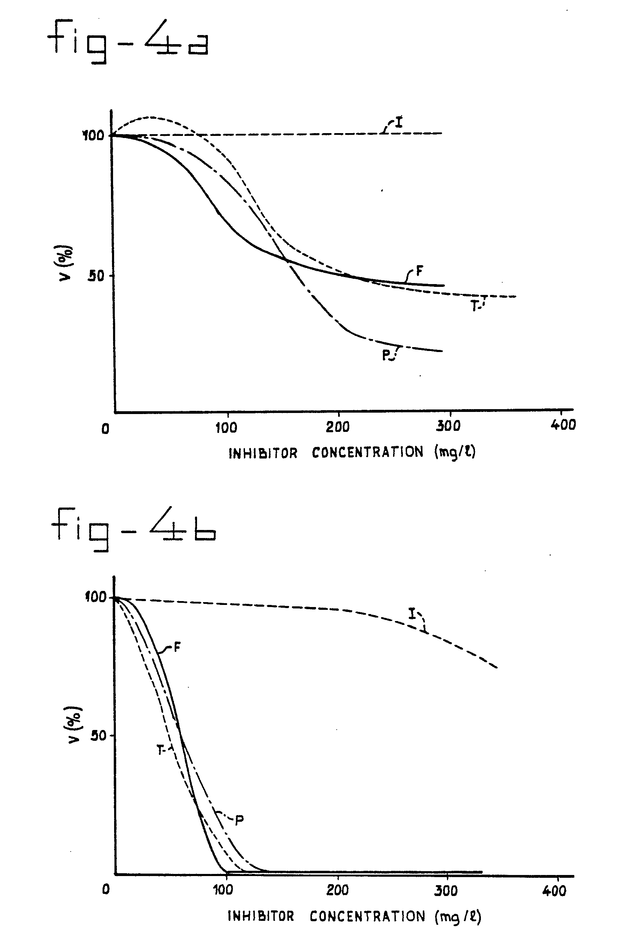

cholestenone is in that case 17,340 l/mol.cm. The effects of adding inhibitors to these reaction mixtures are shown in Figures 3a, 3b, 4a and 4b.

More particularly, figures 3a and 3b illustrate the effect of some inhibitors on Nocardia erythropolis cholesterol oxidase with cholesterol (Figure 3a) and cholest-5-ene-3-one (Figure 3b) as substrates. The assay contained 25% isopropanol. Fenpropimorph (F), tridemorph (T), fenpropidine (P) and imazalil (I) were used as inhibitors.

Figures 4a and 4b illustrate the effect of some inhibitors on Pseudomonas testosteroni cholesterol oxidase with pregnenolone as substrate on the oxidation (Figure 4a) and the total reaction (oxidation and isomerization) (Figure 4b). Fenpropimorph (F), tridemorph (T) fenpropidine (P) and imazalil (I) were used as inhibitors.

From these experiments it appears that the isomerization reaction is considerably inhibited while the oxidation reaction can proceed virtually unhindered. In this manner, the production of 3-keto-$\Delta^5$ steroids can be achieved in a simple manner.

## EXAMPLE II

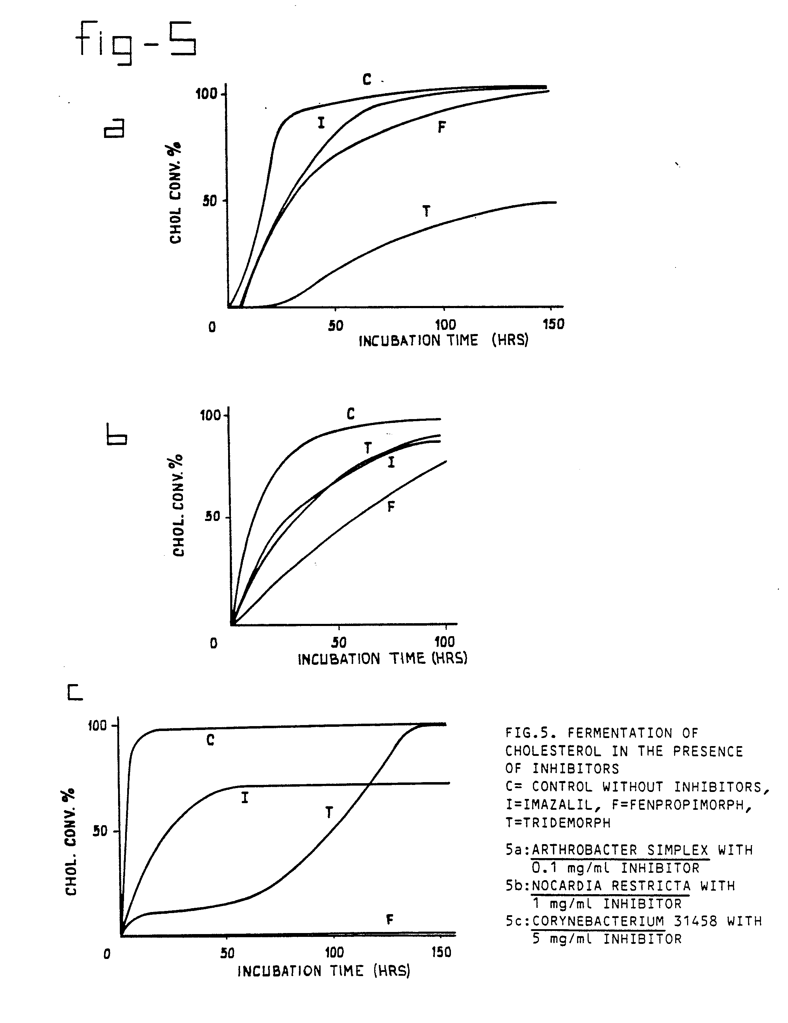

Arthrobacter simplex ATCC 15799, Corynebacterium ATCC 31458, Mycobacterium sp. NRRL-B 3683 and Norcardia restricta ATCC 14884 were cultured overnight in conical flasks with a capacity of 250 ml which contained 50 ml of L medium (see Example III for composition). The following morning, cholesterol in a quantity of 0.1% (w/v) and inhibitors in three concentrations, viz. 0.1, 1 and 5 mg/l were added. At regular time intervals, samples were taken and analysed for the following parameters: pH, cell growth and steroid spectrum. The cholest-4-ene-3-one production of a few conversions is shown in Figure 5. The results of the conversions are listed in Table A below.

Imazalil, which exhibits no inhibiting action on the reaction 2 --→ 3 (Figure 1) was included in the experiment as a control, being a specific, pH-dependent inhibitor. Imazalil usually caused a considerable decrease in pH, namely from 8-9 in the control experiment to a pH of 5-5.5 with 5 mg/l imazalil. The imazalil was used at various pHs with Arthrobacter simplex (pH = 6.0, 6.4 and 7.0) but this had no additional effect on the cell growth and the rate of steroid transformation.

## TABLE A

| inhibitor (mg/l) | Growth 0.1 | Transform. 0.1 | 0.1 | Growth 1 | Transform. 1 | 1 | Growth 5 | Transform. 5 | 5 |
|---|---|---|---|---|---|---|---|---|---|
| time (hrs) | 0-144 | 24 | 72 | 0-144 | 24 | 72 | 0-144 | 24 | 72 |
| **A.simplex** | | | | | | | | | |
| fenpropimorph | A/C | A | C | A/C | S | S | S | D | D |
| tridemorph | A | S | D | A/D | S | S | D | S | D |
| imazalil | D | A | C | S | S | S | S | S | S |
| **C ATCC 31458** | | | | | | | | | |
| fenpropimorph | A/C | C | C | C | A/D | A/D | S | S | S |
| tridemorph | D | C | C | S | A/D | A/D | S | D | A |
| imazalil | S | C | C | S | C | C | S | A | A |
| **N. restricta** | | | | | | | | | |
| fenpropimorph | C | D | C | C | D | A | S/D | D | A/D |
| tridemorph | C | D | C | D | A/D | A | D | D | A/D |
| imazalil | S/D | C | A/D | S | A/D | A | S | D | D |
| **M.sp** | | | | | | | | | |
| fenpropimorph | A/D | D | D | S | S | S | S | S | S |
| tridemorph | D | D | D | S | S | S | S | S | S |
| imazalil | S | S | S | S | S | S | S | S | S |

Key to Table A:

The bacterial growth and cholesterol fermentation in the presence of the inhibitors according to the invention. In the case of Arthrobacter simplex, Corynebacterium ATCC 31458 and Nocardia restricta, the quantity of cholest-4-ene-3-one prepared was compared with the control, and in the case of Mycobacterium sp. NRRL-B 3683, the activity was examined in relation to side-chain detachment. Abbreviations: C = as control, 90 - 100%; A = affected to some extent, 50 - 90%; D = considerably more inhibited, 10 - 50%; S = virtually completely stopped, 0 - 10%; transform. = steroid transformation.

With an inhibitor concentration of 0.1 mg/l, Arthrobacter simplex, Corynebacterium ATCC 31458 and Nocardia restricta reacted more or less similarly to the control as regards cell growth and cholestenone production.

For Arthrobacter simplex and Corynebacterium ATCC 31458, both the growth and the cholest-4-ene-3-one production were considerably suppressed at 1 mg/l and 5 mg/l inhibitor. For Nocardia restricta and Corynebacterium ATCC 31458, the cholest-4-ene-3-one production proceeded in the case of imazalil, in particular with prolonged incubation times at a quantity of 1 and 5 mg/l inhibitor although the growth was affected to a considerable extent. Androst-4-ene-3,17-dione was detected after an incubation time of 120 hours. In general, imazalil , which has a strong negative effect on cell growth, does not inhibit the cholesterol oxidase already present, in the cholest-4-ene-3-one production.

The Mycobacterium strain used proved to be very sensitive to the sterol biosynthesis inhibitors used. Even with a quantity of 0.1 mg/l inhibitor, the cell growth was considerably influenced or even stopped. The same applied to the side chain breakdown activity.

### EXAMPLE III

In this test, the sterol biosynthesis inhibitors tridemorp and fenpropimorph were used as inhibitors according to the invention. The substrates used were cholesterol, sitosterol and 3-cholestanol. As strains, use was made of Nocardia erythropolis IMET and Mycobacterium sp. NRRL-B 3683.

The fermentations were carried out at 30°C in conical flasks with a capacity of 250 ml which contained 50 ml of L medium with the addition of 1 g/l substrate and optionally 0.5 mg/l inhibitor. The L medium contains per litre: yeast extract 5 g, bactotryptone 10 g, NaCl 5 g (pH: 7.0). The steroids were analysed as MO-TMS derivatives by means of a Packard 436 gas chromatograph which contained a 25 m long Chrompack CP Sil 5 CB column.

In the case of Nocardia erythropolis, tridemorph and imazalil have an inhibiting effect on the growth, which effect is less strong in the case of fenpropimorph. If the last-named inhibitor is used, a growth, which is comparable with that in the case of the uninhibited culture, occurs even after approximately 72 hours. The conversion of cholesterol into $\Delta^4$-cholestenone is, however, considerably inhibited by adding inhibitors.

The rate of conversion of cholesterol, sitosterol and 3-cholestanol into androstenedione by Mycobacterium sp. NRRL-B 3683 in the presence of tridemorph is at a level equal to that of fermentations without tridemorph.

### Claims

1. Process for preparing steroids by means of an enzymatic or microbial conversion, characterized in that said conversion is carried out in the presence of a 3-phenylpropylamine or alkylamine compound having the formula 1a or 1b in which $R_1$ represents a group having the formula 2, 3, 4 or 5, $R_2$ represents -O- or -CH$_2$-, $R_3$ represents -CH$_3$ or -H, and $R_4$ represents -CH$_3$ or -H or related compounds having the same mechanism of action, namely the inhibition of $\Delta \xrightarrow{8} \Delta^7$ isomerization, $\Delta^{14}$ reduction, cycloeucalenolobtusifoliol isomerization and $\Delta \xrightarrow{5} \Delta^4$ isomerization.

2. Process according to Claim 1, characterized in that androst-4-ene-3,17-dione and/or androsta-1,4-diene-3,17-dione or androst-5-ene-3,17-dione and/or androsta-1,5-diene-3,17-dione is prepared by means of a microbial conversion of cholesterol, sitosterol, campesterol or stigmasterol or a mixture of said starting substances.

3. Process according to Claim 1 or 2, characterized in that the microbial conversion is carried out in the presence of 0.001-10 g of tridemorph per litre of reaction medium.

4. Process according to any of Claims 1-3 characterized in that the microbial conversion is carried out with strains of the genera Arthrobacterium, Corynebacterium, Nocardia, Pseudomonas, Mycobacterium, Brevibacterium, Schizophyllum and Streptomyces.

5. Process according to Claim 4, characterized in that the microbial conversion is carried out with the strains Arthrobacter simplex ATCC 15799, Corynebacterium ATCC 31458, Mycobacterium sp. NRRL-B 3683 or Nocardia restricta ATCC 14884.

6. Process according to Claim 3 and 5, characterized in that the microbial conversion is carried out by means of the strain Arthrobacter simplex ATCC 15799 or Mycobacterium NRRL-B 3683.

7. Process according to Claim 1, characterized in that 3-keto-$\Delta^5$ steroids are prepared by means of an enzymatic conversion with the aid of microbial cholesterol oxidases in intact cells or in purified form.

8. Process according to Claim 7, characterized in that the cholesterol oxidases originate from or are contained in Nocardia, Corynebacterium, Arthrobacter, Mycobacterium, Pseudomonas, Schizophyllum, Brevibacterium, Rhodococcus and Streptomyces species.

9. Process according to Claim 7, characterized in that said conversions are carried out in the presence of 0.001 - 10 g/l 3-phenylpropylamine or alkylamine compounds having the formula 1a or 1b, wherein $R_1$, $R_2$, $R_3$ and $R_4$ have the meaning stated in Claim 1.

10. Process according to Claim 7, characterized in that fenpropimorph, tridemorph or fenpropidine are used as 3-phenylpropylamine or alkylamine compounds.

**1a.**

cis-(2)

**1b.**

trans-(2)

**2.**

$$CH_3-CH-CH_2-CH_2-CH_2-\overset{*}{C}H-CH_2-CH_2-CH_2-\overset{*}{C}H-$$
$$\quad\quad\;|\quad\quad\quad\quad\quad\quad\;|\quad\quad\quad\quad\quad\quad\quad\;|$$
$$\quad\quad CH_3\quad\quad\quad\quad\quad\;CH_3\quad\quad\quad\quad\quad\;\;CH_3$$

**3.**

$$CH_2-CH_2$$
$$CH_2-CH_2 \quad CH_2-CH-$$
$$CH_2-CH_2 \quad CH_2-CH_2$$
$$CH_2-CH_2$$

**4.**

$$CH_3-\overset{\displaystyle CH_3}{\underset{\displaystyle CH_3}{C}}-\!\!\langle\ \rangle\!\!-CH_2-\overset{*}{C}H-CH_2-$$
$$\quad\quad\quad\quad\quad\quad\quad\quad\quad\quad\quad|$$
$$\quad\quad\quad\quad\quad\quad\quad\quad\quad\quad CH_3$$

**5.**

$$CH_3-CH_2-\overset{\displaystyle CH_3}{\underset{\displaystyle CH_3}{C}}-\!\!\langle\ \rangle\!\!-CH_2-\overset{*}{C}H-CH_2-$$
$$\quad\quad\quad\quad\quad\quad\quad\quad\quad\quad\quad\quad\quad|$$
$$\quad\quad\quad\quad\quad\quad\quad\quad\quad\quad\quad CH_3$$

**6.**

Fig-1

1 CHOLESTEROL
2 $\Delta^5$-CHOLESTENONE
3 $\Delta^4$-CHOLESTENONE
4 $\Delta^{1,4}$-CHOLESTADIENONE
10 $\Delta^4$- PREGNENONE-20-CARBOXYLIC ACID
11 $\Delta^4$- ANDROSTENEDIONE

0 274 147

Fig-2

0 274 147

0 274 147

fig-3a

fig-3b

0 274 147

fig - 4a

fig - 4b

fig-5

FIG.5. FERMENTATION OF
CHOLESTEROL IN THE PRESENCE
OF INHIBITORS
C= CONTROL WITHOUT INHIBITORS,
I=IMAZALIL, F=FENPROPIMORPH,
T=TRIDEMORPH

5a:ARTHROBACTER SIMPLEX WITH
    0.1 mg/ml INHIBITOR
5b:NOCARDIA RESTRICTA WITH
    1 mg/ml INHIBITOR
5c:CORYNEBACTERIUM 31458 WITH
    5 mg/ml INHIBITOR